(19) **Europäisches Patentamt / European Patent Office / Office européen des brevets**

(11) **EP 1 717 319 B1**

(12) **EUROPEAN PATENT SPECIFICATION**

(45) Date of publication and mention
of the grant of the patent:
**18.03.2009 Bulletin 2009/12**

(51) Int Cl.:
*G01N 33/52* (2006.01)          *G01N 33/543* (2006.01)
*C12Q 1/00* (2006.01)

(21) Application number: **06252275.0**

(22) Date of filing: **27.04.2006**

(54) **Method for manufacturing an electrochemical-based analytical test strip with hydrophilicity enhanced metal electrodes**

Verfahren zur Herstellung eines analytischen Teststreifens auf elektrochemischer Basis mit Metallelektroden mit verstärkten hydrophilen Eigenschaften

Procédé de fabrication de bandelette réactive analytique à base électrochimique avec des électrodes de métal au caractère hydrophile amélioré

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.04.2005 US 118265**

(43) Date of publication of application:
**02.11.2006 Bulletin 2006/44**

(73) Proprietor: **Lifescan Scotland Limited**
**Inverness-shire IV2 3ED (GB)**

(72) Inventors:
• **Plotkin, Elliot V.**
**Inverness-shire IV2 3JZ (GB)**
• **Day, Richard Michael**
**Nairn IV12 5RF (GB)**

(74) Representative: **Mercer, Christopher Paul et al**
**Carpmaels & Ransford**
**43-45 Bloomsbury Square**
**London WC1A 2RA (GB)**

(56) References cited:
**EP-A- 0 795 601**          **EP-A- 1 235 068**
**US-A1- 2004 069 657**

• **WILLNER I ET AL: "ELECTRICAL COMMUNICATION BETWEEN ELECTRODES AND NAD(P)+-DEPENDENT ENZYMES USING PYRROLOQUINOLINEQUINONE-ENZYME ELECTRODES IN A SELF- ASSEMBLED MONOLAYER CONFIGURATION: DESIGN OF A NEW CLASS OF AMPEROMETRIC BIOSENSORS" ANALYTICAL CHEMISTRY, AMERICAN CHEMICAL SOCIETY. COLUMBUS, US, vol. 66, no. 9, 1 May 1994 (1994-05-01), pages 1535-1539, XP000446029 ISSN: 0003-2700**
• **KAJIYA Y ET AL: "GLUCOSE SENSITIVITY OF THIOL-MODIFIED GOLD ELECTRODES HAVING IMMOBILIZED GLUCOSE OXIDASE AND 2-AMINOCTHYLFERROCENE" CHEMISTRY LETTERS, CHEMICAL SOCIETY OF JAPAN. TOKYO, JP, vol. 12, 1993, pages 2107-2110, XP001062321 ISSN: 0366-7022**

**Description**

BACKGROUND OF INVENTION

1. Field of the Invention

This invention relates, in general, to analytical devices and, in particular, to electrochemical-based analytical test strips and associated methods.

2. Description of the Related Art

The determination (e.g., detection and/or concentration measurement) of an analyte in a fluid sample is of particular interest in the medical field. For example, it can be desirable to determine glucose, cholesterol, acetaminophen and/or HbAlc concentrations in a sample of a bodily fluid such as urine, blood or interstitial fluid. Such determinations can be achieved using analytical test strips, based on, for example, photometric or electrochemical techniques, along with an associated meter. For example, the OneTouch® Ultra® whole blood testing kit, available from LifeScan, Inc., Milpitas, USA, employs an electrochemical-based analytical test strip for the determination of blood glucose concentration in a whole blood sample.

Typical electrochemical-based analytical test strips employ a plurality of electrodes (e.g., a working electrode and a reference electrode) and an enzymatic reagent to facilitate an electrochemical reaction with an analyte of interest and, thereby, determine the concentration of the analyte. For example, an electrochemical-based analytical test strip for the determination of glucose concentration in a blood sample can employ an enzymatic reagent that includes the enzyme glucose oxidase and the mediator ferricyanide. Further details of conventional electrochemical-based analytical test strips are included in U.S. Patent No. 5,708,247.

EP 1 235 068 discloses a biosensor which is constituted by disposing an electrode system including a working electrode and a counter electrode on a substrate, forming an inorganic particulate-containing layer containing inorganic particulates thereon, and forming a reagent layer containing a reagent thereon.

EP 0 795 601 discloses a biosensor comprising an electrically insulating base plate, an electrode system including a working electrode and a counter electrode provided on the base plate, and a reaction layer containing a hydrophilic polymer, an enzyme and an electron acceptor located over the electrode system.

SUMMARY OF INVENTION

The present invention provides a method for manufacturing a portion of an electrochemical-based test strip, the method comprising forming at least one metal electrode on a surface of an electrically-insulating substrate, the at least one metal electrode having an upper surface; treating the upper surface of the at least one metal electrode with a hydrophilicity-enhancing composition to form a treated upper surface on the metal electrode with hydrophilicity-enhancing chemical moieties thereon; and depositing an enzymatic reagent layer on the treated upper surface of the at least one metal electrode. Wherein the hydrophilicity-enhancing moieties comprise a $SO_2OH$ (sulphonate) group.

Optionally, the forming step includes forming a metal electrode of at least one of gold, palladium, platinum, indium, titanium-palladium alloys and combinations thereof.

Optionally, the forming step includes forming at least one gold metal electrode.

Optionally, the depositing step includes depositing an enzymatic reagent layer that includes a glucose specific enzyme.

Optionally, the treating step employs a chemical reaction of the form:

$$X\text{-}R\text{-}SH + Au \rightarrow X\text{-}R\text{-}S^-Au^+ + \tfrac{1}{2}\,H_2$$

where:

X is a $SO_2OH$ (sulphonate) group;
R is a carbon chain, optionally a carbon chain in the range of $C_1$ to $C_5$;
SH is a thiol group;
Au represents atomic gold; and
$X\text{-}R\text{-}S^-Au^+$ represents the gold metal electrode surface with a hydrophilicity-enhancing moiety thereon.

Optionally, the treating step spontaneously forms hydrophilicity-enhancing chemical moieties on the metal electrode in a non-self-assembled monolayer manner.

Optionally, the treating step employs a hydrophilicity-enhancing composition that includes MESNA.

Optionally, the treating step involves dipping the at least one metal electrode in a hydrophilicity-enhancing composition that includes MESNA to spontaneously form a treated upper surface on the metal electrode with hydrophilicity-enhancing moieties thereon.

Optionally, the forming step employs a photolithographic technique.

BRIEF DESCRIPTION OF DRAWINGS

A better understanding of the features and advantages of the present invention will be obtained by reference to the following detailed description that sets forth illustrative embodiments, in which the principles of the invention are utilized, and the accompanying drawings of which:

FIG. 1 is a simplified exploded perspective view of an electrochemical-based analytical test strip made according to the method of the present invention;

FIG. 2 is a simplified plan view of the patterned conductive layer of the electrochemical-based analytical test strip of FIG. 1;

FIG. 3 is a simplified plan view of a portion of the electrically-insulating substrate, conductive layer and insulating layer of the electrochemical-based analytical test strip of FIG. 1;

FIGs. 4A and 4B are simplified depictions of a chemical sequence for treating a gold metal electrode surface and the resulting gold electrode surface with hydrophilicity-enhancing moieties comprising a $SO_2OH$ group thereon, respectively.

FIG. 5 is a bar chart depicting the water contact angle for a clean gold substrate surface, a clean polyester substrate surface, a clean gold substrate surface treated with MESNA and a clean polyester substrate surface treated with MESNA;

FIG. 6 is a bar chart depicting the water contact angle for a clean gold substrate surface, a clean gold substrate surface treated with MESNA and a clean gold substrate surface treated with MESNA after storage for two weeks;

FIG. 7 is an artist's rendition of a photographic image of a portion of a comparison electrochemical-based analytical test strip with gold electrodes in the absence of hydrophilicity-enhancing moieties comprising a $SO_2OH$ group on the upper surface of the gold electrodes;

FIG. 8 is a chart of current response versus YSI determined glucose concentration for a comparison electrochemical-based analytical test strip with gold metal electrodes in the absence of hydrophilicity-enhancing moieties comprising a $SO_2OH$ group on the upper surface of the gold electrodes;

FIG. 9 is an artist's rendition of a photographic image of a portion of an electrochemical-based analytical test strip with gold metal electrodes according to an exemplary embodiment of the present invention that includes hydrophilicity-enhancing moieties comprising a $SO_2OH$ group on the upper surface of gold metal electrodes;

FIG. 10 is a chart of current response versus YSI determined glucose concentration for an electrochemical-based analytical test strip with gold metal electrodes according to an exemplary embodiment of the present invention that includes hydrophilicity-enhancing moieties comprising a $SO_2OH$ group on the upper surface of the gold metal electrodes; and

FIG. 11 is a flow chart of a process for manufacturing a portion of an electrochemical-based analytical test strip according to an exemplary embodiment of the present invention.

DETAILED DESCRIPTION OF THE INVENTION

An electrochemical-based analytical test strip made according to the method of the present invention includes an electrically-insulating substrate and at least one metal electrode (e.g., a gold metal electrode) disposed on a surface of the electrically-insulating substrate. In addition, the metal electrode has an upper surface with hydrophilicity-enhancing chemical moieties comprising a $SO_2OH$ group thereon and an enzymatic reagent layer disposed on the upper surface. Details, characteristics and benefits of such an electrochemical-based analytical test strip are described below.

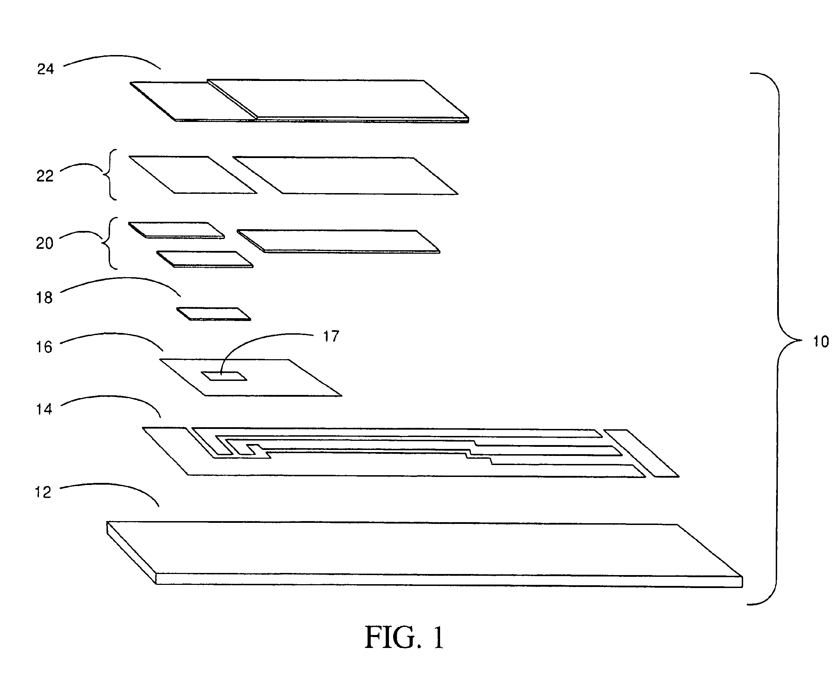

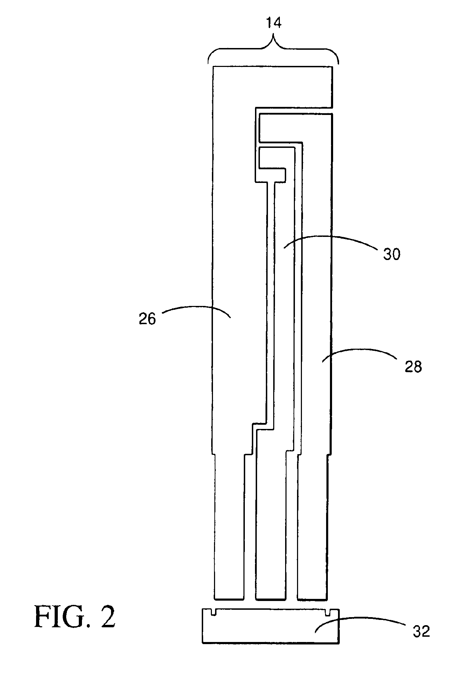

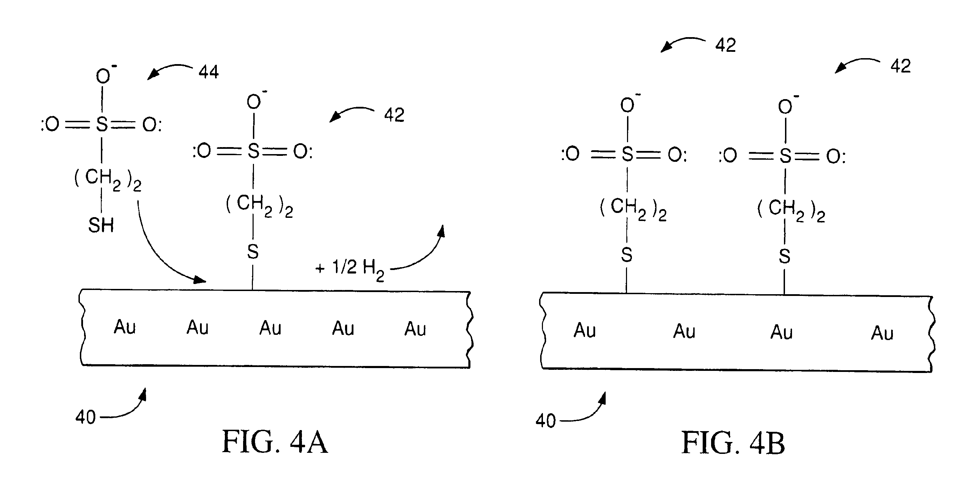

FIG. 1 is a simplified exploded perspective view of an electrochemical-based analytical test strip 10 made according to the method of the present invention. Electrochemical-based analytical test strip 10 includes an electrically-insulating substrate 12, a patterned conductor layer 14, an insulation layer 16 (with electrode exposure window 17 extending therethrough), an enzymatic reagent layer 18, a patterned adhesive layer 20, a hydrophilic layer 22 and a top film 24. As will be described in more detail below with respect to FIGs. 2, 3, 4A and 4B, patterned conductor layer 14 includes three electrodes and at least a portion of each of these electrodes has an upper surface with hydrophilicity-enhancing moieties comprising a $SO_2OH$ group (depicted in FIG. 4B) thereon.

Electrically-insulating substrate 12 can be any suitable electrically-insulating substrate known to one skilled in the art including, for example, a nylon substrate, polycarbonate substrate, a polyimide substrate, a polyvinyl chloride substrate, a polyethylene substrate, a polypropylene substrate, a glycolated polyester (PETG) substrate, or a polyester substrate. The electrically-insulating substrate can have any suitable dimensions including, for example, a width dimension of about 5 mm, a length dimension of about 27 mm and a thickness dimension of about 0.5 mm.

Insulation layer 16 can be formed, for example, from a screen printable insulating ink. Such a screen printable insulating ink is commercially available from Ercon of Wareham, Massachusetts U.S.A. under the name "Insulayer." Patterned adhesive layer 20 can be formed, for example, from a screen-printable pressure sensitive adhesive commercially available from Apollo Adhesives, Tamworth, Staffordshire, UK.

Hydrophilic layer 22 can be, for example, a clear film with hydrophilic properties that promote wetting and filling of electrochemical-based analytical test strip 10 by a fluid sample (e.g., a whole blood sample). Such clear films are commercially available from, for example, 3M of Minneapolis, Minnesota U.S.A. Top film 24 can be, for example, a clear film overprinted by black decorative ink. A suitable clear film is commercially available from Tape Specialities, Tring, Hertfordshire, UK.

Enzymatic reagent layer 18 can include any suitable enzymatic reagents, with the selection of enzymatic reagents being dependent on the analyte to be determined. For example, if glucose is to be determined in a blood sample, enzymatic reagent layer 18 can include oxidase or glucose dehydrogenase along with other components necessary for functional operation. Further details regarding enzymatic reagent layers, and electrochemical-based analytical test strips in general, are in U.S. Patent No. 6,241,862.

Electrochemical-based analytical test strip 10 can be manufactured, for example, by the sequential aligned formation of patterned conductor layer 14, insulation layer 16 (with electrode exposure window 17 extending therethrough), enzymatic reagent layer 18, patterned adhesive layer 20, hydrophilic layer 22 and top film 24 onto electrically-insulating substrate 12. Any suitable techniques known to one skilled in the art can be used to accomplish such sequential aligned formation, including, for example, screen printing, photolithography, photogravure, chemical vapour deposition and tape lamination techniques.

FIG. 2 is a simplified plan view of patterned conductive layer 14 of electrochemical-based analytical test strip 10. Patterned conductive layer 14 includes a counter electrode 26 (also referred to as a reference electrode), a first working electrode 28, a second working electrode 30 and a contact bar 32. Although electrochemical-based analytical test strip 10 is depicted as including three electrodes, embodiments of electrochemical-based analytical test strips made according to the method of the present invention can include any suitable number of electrodes.

Counter electrode 26, first working electrode 28 and second working electrode 30 can be formed of any suitable electrode metal including, for example, gold, palladium, platinum, indium and titanium-palladium alloys. The formation of such metal electrodes typically results in a metal electrode with a smooth, albeit hydrophobic, surface.

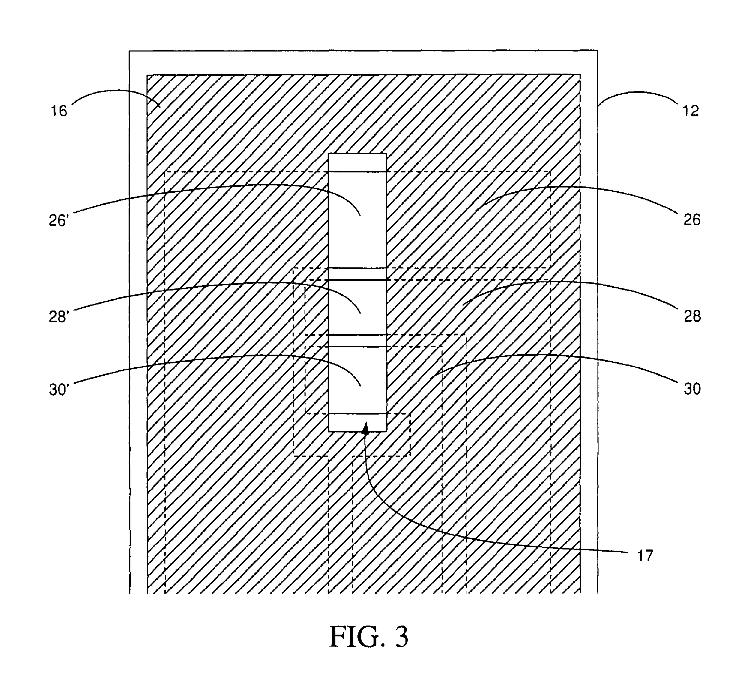

FIG. 3 is a simplified plan view of a portion of electrically-insulating substrate 12, patterned conductive layer 14 and insulating layer 16 (shaded with cross-hatching) of electrochemical-based analytical test strip 10. Electrode exposure window 17 of insulation layer 16 exposes a portion of counter electrode 26, a portion of first working electrode 28 and a portion of second working electrode 30, namely counter electrode exposed portion 26', first working electrode exposed portion 28' and second working electrode exposed portion 30'. During use, a fluid sample is communicated to electrode exposure window 17 and thereby operatively contacted with counter electrode exposed portion 26', first working electrode exposed portion 28' and second working electrode exposed portion 30'.

Counter electrode exposed portion 26', first working electrode exposed portion 28' and second working electrode exposed portion 30' can have any suitable dimensions. For example, counter electrode exposed portion 26' can have a width dimension of about 0.72 mm and a length dimension of about 1.6 mm, while first working electrode exposed portion 28' and second working electrode exposed portion 30' can each have a width dimension of about 0.72 mm and a length dimension of about 0.8 mm.

Following formation of insulation layer 16, patterned conductive layer 14, and the disposition of hydrophilicity-enhancing moieties comprising a $SO_2OH$ group on the counter electrode exposed portion 26', the first working electrode exposed portion 28' and the second working electrode exposed portion 30', enzymatic reagent layer 18 is applied over counter electrode exposed portion 26', first working electrode exposed portion 28' and second working exposed portion 30'. Details regarding the use of such electrodes, electrode exposed portions and enzymatic reagent layers for the determination of the concentrations of analytes in a fluid sample, albeit without the hydrophilicity-enhancing moieties comprising a $SO_2OH$ group described in this disclosure, are in U.S. Patent No. 6,733,655.

During use of electrochemical-based analytical test strip 10 to determine an analyte concentration in a fluid sample (e.g., blood glucose concentration in a whole blood sample), counter electrode 26, first working electrode 28 and second working electrode 30 are employed to monitor an electrochemical reaction induced current of interest. The magnitude of such a current can then be correlated with the amount of analyte present in the fluid sample under investigation.

The current measured by a working electrode is governed by the following simplified equation:

$$i = nFAJ \qquad\qquad Eq.\ 1$$

where:

*i* is a measured current;
*n* is a number of electrons generated during the reaction;
F is the Faraday constant;
A is an area of the electrode at which a reaction occurs (also referred to as the active surface area of the electrode); and
J is the flux of a species of interest to the electrode.

Based on equation 1 above, a reliable and accurate determination (e.g., quantification) of an analyte concentration in a fluid sample requires knowledge of the area of the working electrode at which the reaction occurs. It has been determined that the sensing area of an electrode in an electrochemical-based analytical test strip is dependent on the uniformity and adherence of an enzymatic reagent layer to the electrode throughout manufacturing and during use. In addition, it has been determined that employing a metal electrode with hydrophilicity-enhancing moieties comprising a $SO_2OH$ group thereon improves the uniformity and adherence of enzymatic reagent layers and, thus, the reproducibility and accuracy of results obtained with electrochemical-based analytical test strips that employ such metal electrodes.

FIGs. 4A and 4B are simplified depictions of a chemical sequence for treating a gold metal electrode surface 40 and the resulting gold metal electrode surface with hydrophilicity-enhancing moieties 42 thereon, respectively. FIG. 4A depicts the manner in which gold metal surface 40 is exposed to a hydrophilicity-enhancing composition 44 to produce hydrophilicity-enhancing moietie 42 comprising a $SO_2OH$ group and liberate hydrogen.

The reaction that occurs between a gold metal electrode surface and the thiol (-SH) group of hydrophilicity-enhancing composition 44 is described by a general reaction sequence of the form:

$$\text{X-R-SH} + \text{Au} \rightarrow \text{X-R-S}^-\text{Au}^+ + \tfrac{1}{2}\,\text{H}_2 \qquad\qquad Seq.\ 1$$

where:

X is a $SO_2OH$ (sulphonate) group ;
R is a carbon chain from, for example, $C_1$ to $C_5$;
SH is a thiol group;
Au represents atomic gold; and
X-R-S⁻Au⁺ represents a gold metal electrode surface with a hydrophilicity-enhancing moiety comprising a $SO_2OH$ group thereon.

In sequence 1 above, R can be beneficially limited to the range of $C_1$ to $C_5$ to provide a hydrophilicity-enhancing composition that is soluble, yet avoids the formation of self-assembled monolayers on the gold metal electrode surface. Self-assembled monolayers of hydrophilicity-enhancing moieties comprising a $SO_2OH$ group need not necessarily be avoided, but their formation is difficult to control, often slow and can require an electrode surface that is "atomically" clean. The manufacturing of such self-assembled monolayers is, therefore, more difficult than the non-self-assembled disposition of hydrophilicity-enhancing moieties comprising a $SO_2OH$ group that occurs spontaneously by dip coating an electrode surface with a MESNA solution as described elsewhere in this disclosure.

Furthermore, the thiol group (also referred to as a "tail" group) enables a conjugation between the gold metal electrode surface and the hydrophilicity-enhancing composition to occur. In addition, the polar, positively charged or negatively charged side group "X" (also referred to as a "head" group) provides for a hydrophilic interaction with an enzymatic reagent layer, thereby improving the uniformity and adherence of the enzymatic reagent layer to the metal electrode upper surface. The head group is a $SO_2OH$ (sulphonate) group.

As noted above, the length of the "R" group (also referred to as a "spacer chain") is a factor in determining whether or not the hydrophilicity-enhancing moieties are disposed on the electrode surface as a self-assembled monolayer.

Although FIGs. 4A and 4B and sequence 1 are illustrated for the circumstance of a gold metal electrode surface, once apprised of the present disclosure one of ordinary skill in the art will recognize that other metal electrode surfaces can also be beneficially treated to dispose hydrophilicity-enhancing moieties comprising a $SO_2OH$ group thereon.

Enzymatic reagents are formulated such that they readily mix with common fluid samples (such as a whole blood or other bodily fluid sample) and, therefore, typically consist of components that are readily soluble in aqueous solutions. It has been determined that such components have an affinity for hydrophilic or at least amphiphilic surfaces.

A variety of metal electrode surfaces are naturally hydrophobic. In other words, such metal electrode surfaces tend to repel water, aqueous solutions, and solutions with significant hydrophilic component content (such as enzymatic reagents). However, it has been determined that such metal electrode surfaces can be rendered more hydrophilic (i.e., be hydrophilically-enhanced) by treating the metal electrodes surfaces with a hydrophilicity-enhancing composition that disposes hydrophilicity-enhancing moieties comprising a $SO_2OH$ group on the metal electrode surface.

Examples of hydrophilicity-enhancing compositions are compositions that contain 2-mercaptoethanesulphonic acid (MESNA), 3-mercaptopropanesulphonic acid, 2,3-dimercaptopropanesulphonic acid and its homologues, bis-(2-sulphoethyl)disulphide, bis-(3-sulphopropyl)disulphide . When such hydrophilicity-enhancing compositions include a compound with a sulphonate moiety (e.g., MESNA) the adhesion of a enzymatic reagent layer to the upper surface of a metal electrode is particularly enhanced.

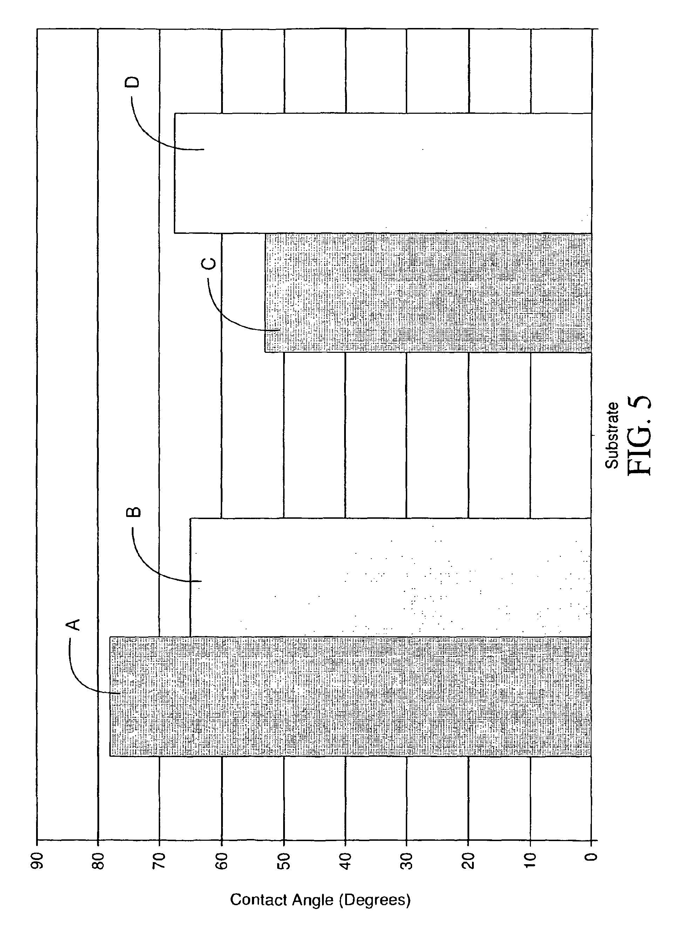

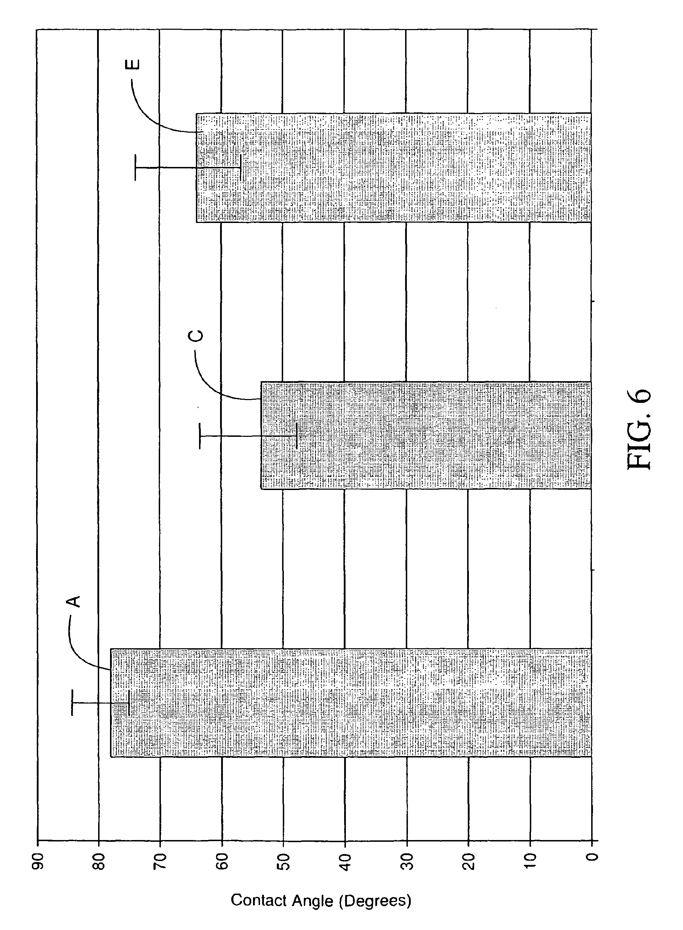

FIG. 5 is a bar chart depicting the water contact angle for clean gold substrate surface (A), a clean polyester substrate surface (B), a clean gold substrate surface treated with MESNA (C) and a clean polyester substrate surface treated with MESNA (D). FIG. 6 is a bar chart depicting the water contact angle for a clean gold substrate surface (A, as in FIG. 5), a clean gold substrate surface treated with MESNA (C, as in FIG. 5) and a clean gold substrate surface treated with MESNA after storage for two weeks (E). The MESNA treatment reflected in FIGs. 5 and 6 was a 5 minute exposure to a MESNA composition consisting of 4g/L of MESNA in water.

As depicted in FIG. 5, the treatment of a clean polyester substrate surface with MESNA did not significantly alter the hydrophilicity of the clean polyester substrate as evidenced by water contact angle. The difference in the measured water contact angles B and D was the within 5%. However, the data of FIG. 5 indicates that the treatment of a clean gold substrate surface with MESNA significantly alters (i.e., enhances) the hydrophilicity of the surface as evidenced by water contact angle. The clean gold substrate surface had a water contact angle of approximately 78 degrees, following treatment with MESNA, the water contact angle was approximately 52 degrees. It is postulated, without being bound, that such a reduction in water contact angle, and therefore increase in hydrophilicity, improves the uniformity and adhesion of enzymatic reagent layers to such treated gold surface. In other words, the treated gold substrate surface, which has hydrophilicity-enhancing moieties comprising a $SO_2OH$ group thereon, will exhibit improved uniformity and adherence with respect to enzymatic reagent layers. In addition, the data of FIG. 6 indicate that the reduction in water contact angle persists after two weeks of storage. Such persistence in enhanced hydrophilicity is beneficial with respect to easing manufacturing time constraints.

Table 1 below lists the water contact angle of gold substrate surfaces that had received various treatments. For treatments 1-15 of Table 1, cleaned gold substrates were exposed to MESNA solutions as indicated in the Table. Treatment 16 consisted of cleaning a gold substrate surface but no exposure to MESNA and treatment 17 involved no cleaning or exposure to MESNA. The data of Table 1 indicate that a significant reduction in water contact angle and, thus, enhancement in hydrophilicity and enzymatic reagent layer adhesion and uniformity, can be achieved with an exposure to MESNA for a time period as short as 1 minute. The data of Table 1, therefore, indicate that the manufacturing of metal electrodes with hydrophilicity-enhancing moieties on their upper surfaces could be accomplished using continuous web-based processes (such as the processes described in WO 01/73109, which is hereby incorporated in full by reference) that have been modified to include a metal electrode upper surface treatment module.

**Table 1**

| Treatment # | MESNA Concentration (g/L) | Time (min) | Average Water Contact Angle (degrees) |
| --- | --- | --- | --- |
| 1 | 16 | 1 | 41 |
| 2 | 16 | 2 | 52 |
| 3 | 16 | 5 | 49 |
| 4 | 16 | 10 | 50 |
| 5 | 16 | 15 | 52 |
| 6 | 4 | 1 | 48 |
| 7 | 4 | 2 | 65 |
| 8 | 4 | 5 | 53 |
| 9 | 4 | 10 | 63 |
| 10 | 4 | 15 | 55 |

(continued)

| Treatment # | MESNA Concentration (g/L) | Time (min) | Average Water Contact Angle (degrees) |
|---|---|---|---|
| 11 | 1 | 1 | 60 |
| 12 | 1 | 2 | 54 |
| 13 | 1 | 5 | 69 |
| 14 | 1 | 10 | 64 |
| 15 | 1 | 15 | 58 |
| 16 | Cleaned | - | 78 |
| 17 | Not cleaned | - | 79 |

Comparative Example

To demonstrate characteristics and benefits of electrochemical-based analytical test strips according to embodiments made by the method of the present invention, a comparison between an electrochemical-based analytical test strip with gold electrodes in the absence of hydrophilicity-enhancing moieties (i.e., a comparison electrochemical-based analytical test strip) and an electrochemical-based analytical test strip with gold metal electrodes according to an exemplary embodiment made by the method of the present invention was undertaken.

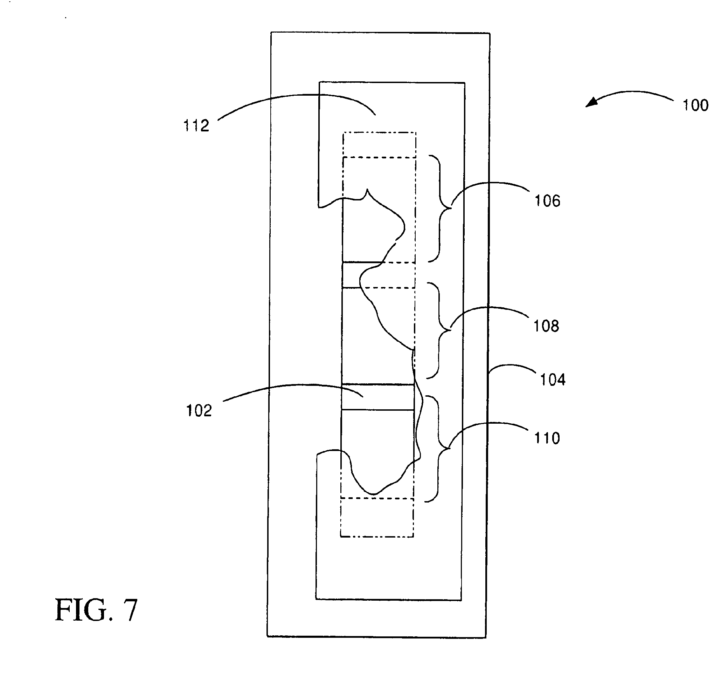

FIG. 7 is an artist's rendition of a photographic image of a portion 100 of an electrochemical-based analytical test strip with gold electrodes in the absence of hydrophilicity enhancing moieties on the upper surface of the gold electrodes. FIG. 7 depicts portion 100 prior to the application of a blood sample thereto. Portion 100 includes an electrically-insulating substrate 102, an insulation layer 104, counter electrode exposed portion 106, first working electrode exposed portion 108, second working electrode exposed portion 110 and enzymatic reagent layer 112. The composition of enzymatic reagent layer 112 and the method by which it was applied are described in U.S. Patent No. 5,708,247.

As is evident from FIG. 7, enzymatic reagent layer 112 exhibits significant nonuniformity over counter electrode exposed portion 106, first working electrode exposed portion 108, and second working electrode exposed portion 110, thus indicating a lack of adherence thereto. Such a lack of uniformity and/or adherence is postulated to be a contributor to unreliable and inaccurate electrochemical-based analytical test strip results. In addition, it has been determined that enzymatic reagent layers disposed on an electrode surface in the absence of hydrophilicity-enhancing moieties are easily damaged during physical manipulation that occurs in conventional test strip manufacturing processes and can separate from the electrode surface upon exposure to a fluid sample.

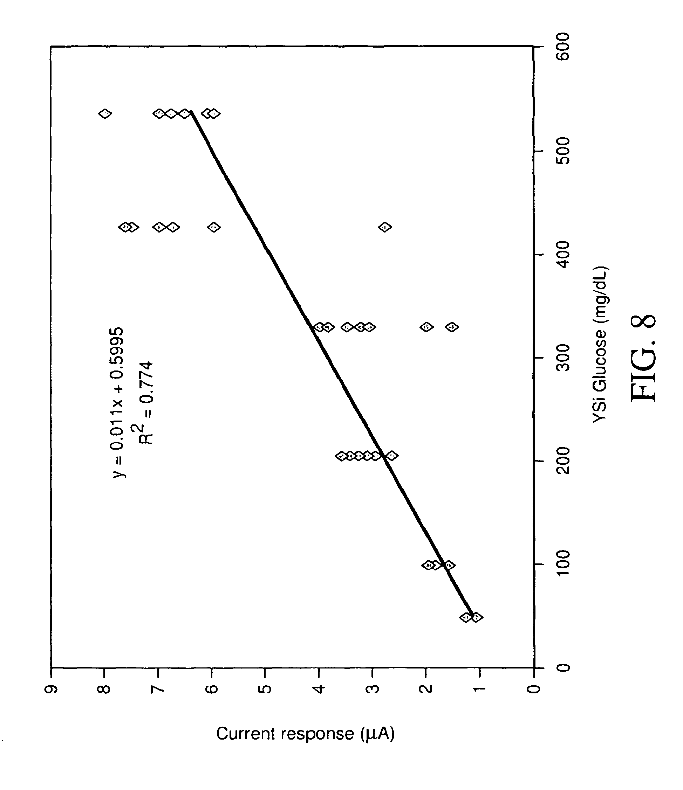

FIG. 8 is a chart of current response versus YSI determined glucose concentration for a comparison electrochemical-based analytical test strip with gold metal electrodes in the absence of hydrophilicity-enhancing moieties on the upper surface of the gold electrodes (i.e., comparison electrochemical-based analytical test strips corresponding effectively to the depiction of FIG. 7). The best fit line and $R^2$ value for the data of FIG. 8 are indicated on the chart. The data and $R^2$ value of FIG. 8 are an indication of the repeatability and accuracy of measurements made with the comparison electrochemical-based analytical test strips with gold electrodes.

As noted above with respect to FIG. 7, enzymatic reagent layer 112 exhibited a lack of uniformity and adherence when employed with a gold metal electrode. It is postulated that such a lack of uniformity and adherence will lead to inaccuracies and lack of measurement repeatability as it adversely and unpredictably affects the sensing area of the working electrodes.

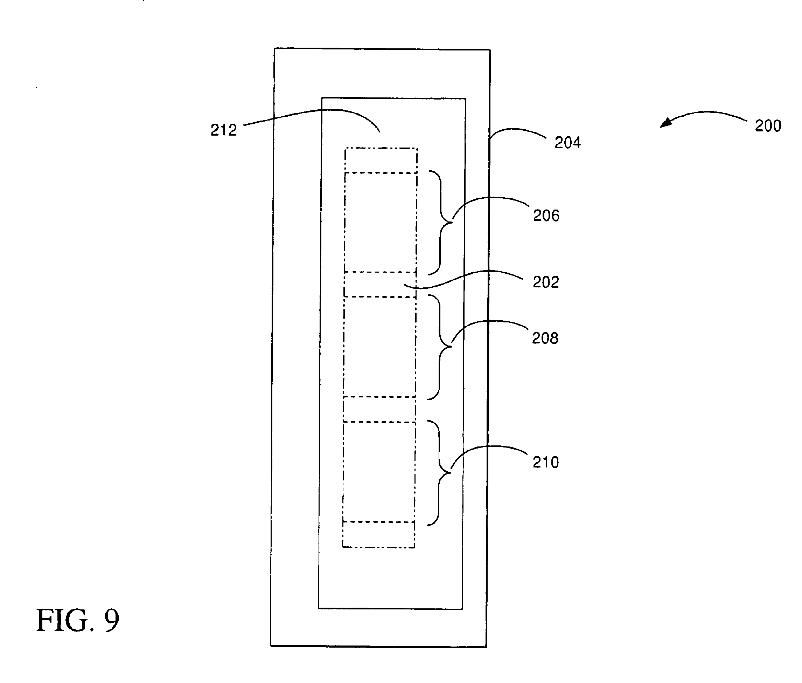

FIG. 9 is an artist's rendition of a photographic image of a portion 200 of an electrochemical-based analytical test strip hydrophilicity-enhancing moieties disposed on the upper surface of gold electrodes. FIG. 9 depicts portion 200 prior to the application of a blood sample thereto. Portion 200 includes an electrically-insulating substrate 202, an insulation layer 204, counter electrode exposed portion 206, first working electrode exposed portion 208, second working electrode exposed portion 210 and enzymatic reagent layer 212. The composition of enzymatic reagent layer 212 and the method by which it was applied are described in U.S. Patent No. 5,708,247. FIG. 9 indicates that enzymatic reagent layer 212 is uniform and fully adhered to counter electrode exposed portion 206, first working electrode exposed portion 208, second working electrode exposed portion 210. In addition, it was determined that enzymatic reagent layers disposed on an electrode surface with hydrophilicity-enhancing moieties were robust to physical manipulation that occurs in conventional test strip manufacturing processes.

Hydrophilicity-enhancing moieties were disposed on counter electrode exposed portion 206, first working electrode exposed portion 208, second working electrode exposed portion 210 by submerging them in a 4g/L aqueous solution of MESNA for 2 minutes followed by a water rinse. This exposure occurred prior to the application of enzymatic reagent layer 212.

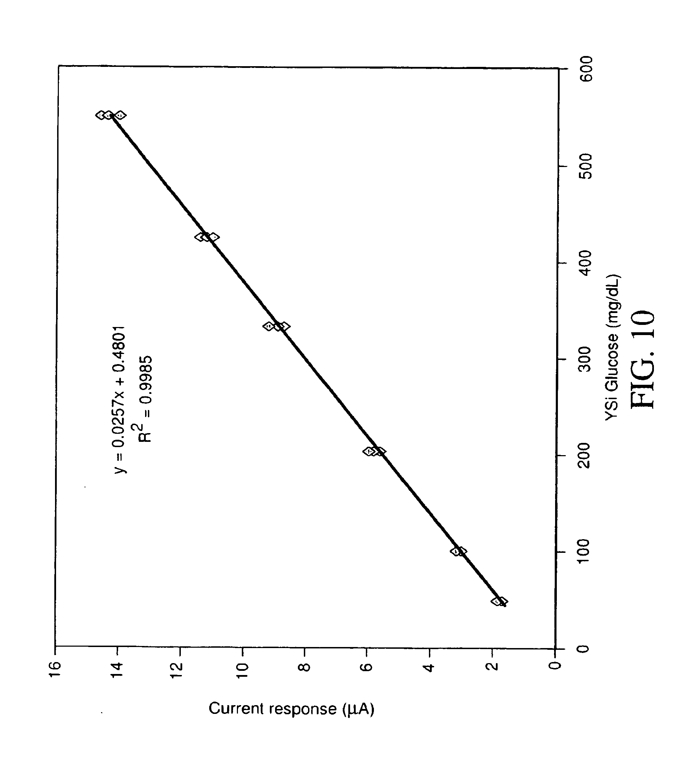

FIG. 10 is a chart of current response versus YSI determined glucose concentration for an electrochemical-based analytical test strip with gold metal electrodes that have hydrophilicity-enhancing moieties on their upper surface of the gold electrodes (i.e., electrochemical-based analytical test strips corresponding effectively to the depiction of FIG. 9). The best fit line and $R^2$ value for the data of FIG. 10 are indicated on the chart. The data and $R^2$ value of FIG. 10 are an indication of the repeatability and accuracy of measurements made with the electrochemical-based analytical test strip with gold electrodes.

A comparison of FIGs. 10 and 8 indicates that the repeatability and accuracy of electrochemical-based analytical test strips that employ a metal electrode with hydrophilicity-enhancing moieties on an upper surface of the metal electrode are superior to a comparison electrochemical-based analytical test strip with metal electrodes in the absence of such hydrophilicity-enhancing moieties. For example, the $R^2$ for the date of FIG. 10 is 0.9985, a significant improvement over the $R^2$ value of 0.774 for the data of FIG. 8.

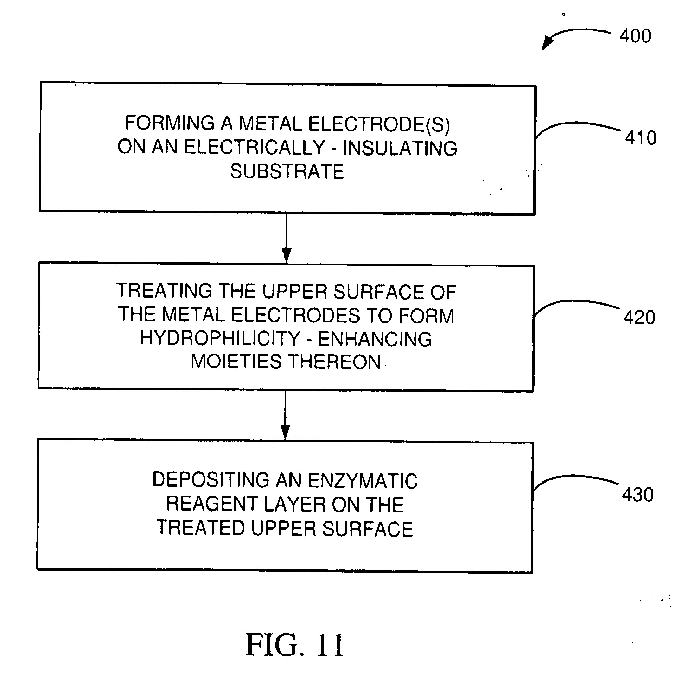

FIG. 11 is a flow chart of a process 400 for manufacturing a portion of an electrochemical-based analytical test strip according to an exemplary embodiment of the present invention. Process 400 includes forming at least one metal electrode (e.g., a gold metal, palladium metal or platinum metal electrode) on a surface of an electrically-insulating substrate with the at least one metal electrode having an upper surface, as set forth in step 410.

Subsequently, the upper surface of each of the at least one metal electrodes is treated with a hydrophilicity-enhancing composition to form a treated upper surface of the metal electrode with hydrophilicity-enhancing chemical moieties thereon, as set forth in step 420. The treatment can be accomplished using, for example, any suitable treatment technique including dip coating techniques, spray coating techniques, and inkjet coating techniques. Any suitable hydrophilicity-enhancing composition can be employed including those described above with respect to electrochemical-based analytical test strips according to the present invention.

The following two examples are illustrate, in a non-limiting manner, treatment technique sequences that can be employed in treatment step 420 of process 400:

Treatment Example 1

(a) clean upper surface of the metal electrode(s) by placing them in a 2% v/v aqueous solution of degreasant (e.g. Micro-90®) for 2 minutes at room temperature.
(b) Rinse the metal electrodes with water to remove excess degreasant.
(c) Dip the metal electrodes into a 4g/L aqueous solution of MESNA) for two minutes.
(d) Rinse the metal electrodes with water to remove excess aqueous solution.
(e) Dry the metal electrodes in a clean environment.

Treatment Example 2

(a) Place the metal electrodes into an ultrasonic bath with an aqueous solution containing 2% v/v degreasant (e.g. Micro-90®) and 4g/L MESNA).
(b) Sonicate the two minutes in an ultrasonic bath at a temperature of 50 °C.
(c) Rinse the metal electrodes with water to remove excess degreasant and MESNA.
(d) Dry the metal electrodes.

Thereafter, at step 430 of process 400, an enzymatic reagent layer is applied to the treated upper surface of the at least one metal electrode.

It should be understood that various alternatives to the embodiments of the invention described herein may be employed in practicing the invention. It is intended that the following claims define the scope of the invention and that structures and methods within the scope of these claims and their equivalents be covered thereby.

**Claims**

1. A method for manufacturing a portion of an electrochemical-based test strip, the method comprising:

　　　forming at least one metal electrode on a surface of an electrically-insulating substrate, the at least one metal electrode having an upper surface;

treating the upper surface of the at least one metal electrode with a hydrophilicity-enhancing composition to form a treated upper surface on the metal electrode with hydrophilicity-enhancing chemical moieties thereon; and depositing an enzymatic reagent layer on the treated upper surface of the at least one metal electrode **characterised in that** the hydrophilicity-enhancing moieties comprise a $SO_2OH$ (sulphonate) group.

2. The method of claim 1, wherein the forming step includes forming a metal electrode of at least one of gold, palladium, platinum, indium, titanium-palladium alloys and combinations thereof.

3. The method of claim 2, wherein the forming step includes forming at least one gold metal electrode.

4. The method of claim 3, wherein the treating step employs a chemical reaction of the form:

$$X\text{-}R\text{-}SH + Au \rightarrow X\text{-}R\text{-}S^-Au^+ + \tfrac{1}{2}H_2$$

where:

X is a $SO_2OH$ (sulphonate) group;
R is a carbon chain;
SH is a thiol group;
Au represents atomic gold; and
$X\text{-}R\text{-}S^-Au^+$ represents the gold metal electrode surface with a hydrophilicity-enhancing moiety thereon.

5. The method of claim 4, wherein R is a carbon chain in the range of $C_1$ to $C_5$.

6. The method of any one of claims 1 to 5, wherein the treating step spontaneously forms hydrophilicity-enhancing chemical moieties on the metal electrode in a non-self-assembled monolayer manner.

7. The method of any one of claims 1 to 6, wherein the depositing step includes depositing an enzymatic reagent layer that includes a glucose specific enzyme.

8. The method of any one of claims 1 to 7, wherein the treating step employs a hydrophilicity-enhancing composition that includes MESNA (2-mercaptoethane sulphonic acid).

9. The method of claim 8, wherein the treating step involves dipping the at least one metal electrode in a hydrophilicity-enhancing composition that includes MESNA to spontaneously form a treated upper surface on the metal electrode with hydrophilicity-enhancing moieties thereon.

10. The method of any one of claims 1 to 9, wherein the forming step employs a photolithographic technique.

**Patentansprüche**

1. Verfahren zur Herstellung eines Teils eines Teststreifens auf elektrochemischer Basis, wobei das Verfahren umfasst:

Ausbilden wenigstens einer Metallelektrode auf einer Oberfläche eines elektrisch isolierenden Substrats, wobei die wenigstens eine Metallelektrode eine obere Oberfläche aufweist;
Behandeln der oberen Oberfläche der wenigstens einen Metallelektrode mit einer die Hydrophilie verstärkenden Zusammensetzung, um eine behandelte obere Oberfläche auf der Metallelektrode mit die Hydrophilie verstärkenden chemischen Einheiten darauf zu bilden; und
Abscheiden einer enzymatischen Reagensschicht auf der behandelten oberen Oberfläche der wenigstens einen Metallelektrode,
**dadurch gekennzeichnet, dass** die die Hydrophilie verstärkenden Einheiten eine $SO_2OH$(Sulfonat)-Gruppe umfassen.

2. Verfahren nach Anspruch 1, wobei der Schritt des Ausbildens das Ausbilden einer Metallelektrode aus wenigstens einem von Gold, Palladium, Platin, Indium, Titan-Palladium-Legierungen und Kombinationen davon einschließt.

3. Verfahren nach Anspruch 2, wobei der Schritt des Ausbildens das Ausbilden wenigstens einer Gold-Metallelektrode einschließt.

4. Verfahren nach Anspruch 3, wobei der Schritt des Behandelns eine chemische Reaktion der Form einsetzt:

$$X\text{-}R\text{-}SH + Au \rightarrow X\text{-}R\text{-}S^-Au^+ + \tfrac{1}{2}\,H_2$$

worin :

X eine $SO_2OH$(Sulfonat)-Gruppe ist;
R eine Kohlenstoffkette ist;
SH eine Thiolgruppe ist;
Au für atomares Gold steht; und
$X\text{-}R\text{-}S\text{-}Au^+$ für die Gold-Metallelektrodenoberfläche mit einer die Hydrophilie verstärkenden Einheit darauf steht.

5. Verfahren nach Anspruch 4, wobei R eine Kohlenstoffkette im Bereich von $C_1$ bis $C_5$ ist.

6. Verfahren nach einem der Ansprüche 1 bis 5, wobei der Schritt des Behandelns spontan die Hydrophilie verstärkende chemische Einheiten auf der Metallelektrode in einer Nicht-Selbstbildungs-Monoschichtweise bildet.

7. Verfahren nach einem der Ansprüche 1 bis 6, wobei der Schritt des Abscheidens das Abscheiden einer enzymatischen Reagensschicht einschließt, die ein glucosespezifisches Enzym einschließt.

8. Verfahren nach einem der Ansprüche 1 bis 7, wobei der Schritt des Behandelns eine die Hydrophilie verstärkende Zusammensetzung einsetzt, die MESNA (2-Mercaptoethansulfonsäure) einschließt.

9. Verfahren nach Anspruch 8, wobei der Schritt des Behandelns das Eintauchen der wenigstens einen Metallelektrode in eine die Hydrophilie verstärkende Zusammensetzung mit sich bringt, die MESNA einschließt, um spontan eine behandelte obere Oberfläche auf der Metallelektrode mit die Hydrophilie verstärkenden Einheiten darauf zu bilden.

10. Verfahren nach einem der Ansprüche 1 bis 9, wobei der Schritt des Ausbildens eine Photolithographietechnik einsetzt.

**Revendications**

1. Méthode de fabrication d'une partie d'une bandelette réactive à base électrochimique, la méthode consistant à :

■ former au moins une électrode métallique sur une surface d'un substrat électriquement isolant, l'au moins une électrode métallique ayant une surface supérieure ;
■ traiter la surface supérieure de l'au moins une électrode métallique avec une composition améliorant le caractère hydrophile pour former une surface supérieure traitée sur l'électrode métallique comprenant des groupes chimiques améliorant le caractère hydrophile ; et
■ déposer une couche de réactif enzymatique sur la surface supérieure traitée de l'au moins une electrode métallique,

**caractérisée en ce que** les groupes améliorant le caractère hydrophile comprennent un groupe $SO_2OH$ (sulfonate).

2. Méthode selon la revendication 1, dans laquelle l'étape de formation comprend la formation d'une électrode métallique en au moins un des alliages d'or, palladium, platine, indium, titane-palladium et combinaisons de ceux-ci.

3. Méthode selon la revendication 2, dans laquelle l'étape de formation comprend la formation d'au moins une électrode en métal en or.

4. Méthode selon la revendication 3, dans laquelle l'étape de traitement emploie une réaction chimique de la forme

$$X\text{-}R\text{-}SH + Au \rightarrow X\text{-}R\text{-}S^-Au^+ + \tfrac{1}{2}\,H_2$$

où :

X est un groupe SO$_2$OH (sulfonate) ;
R est une chaîne carbonée ;
SH est un groupe thiol ;
Au représente l'or atomique ; et
X-R-S⁻Au⁺ représente la surface d'électrode métallique en or comprenant un groupe améliorant le caractère hydrophile.

**5.** Méthode selon la revendication 4, dans laquelle R est une chaîne carbonée dans la plage de C$_1$ à C$_5$.

**6.** Méthode selon l'une quelconque des revendications 1 à 5, dans laquelle l'étape de traitement forme spontanément des groupes améliorant le caractère hydrophile sur l'électrode métallique à la manière d'une monocouche non auto-assemblée.

**7.** Méthode selon l'une quelconque des revendications 1 à 6, dans laquelle l'étape de dépôt inclut le dépôt d'une couche de réactif enzymatique qui inclut un enzyme spécifique du glucose.

**8.** Méthode selon l'une quelconque des revendications 1 à 7, dans laquelle l'étape de traitement emploie une composition améliorant le caractère hydrophile qui inclut du MESNA (acide 2-mercaptoéthane sulfonique).

**9.** Méthode selon la revendication 8, dans laquelle l'étape de traitement implique l'immersion de l'au moins une électrode métallique dans une composition améliorant le caractère hydrophile qui inclut du MESNA pour former spontanément une surface supérieure traitée sur l'électrode métallique comportant des groupes améliorant le caractère hydrophile.

**10.** Méthode selon l'une quelconque des revendications 1 à 9, dans laquelle l'étape de formation emploie une technique photolithographique.

FIG. 1

FIG. 2

FIG. 3

EP 1 717 319 B1

FIG. 4B

FIG. 4A

FIG. 5

FIG. 6

EP 1 717 319 B1

100

106
108
104
110

112

102

FIG. 7

18

FIG. 8

FIG. 9

EP 1 717 319 B1

FIG. 10

```
┌─────────────────────────────────────────┐
│   FORMING A METAL ELECTRODE(S)           │
│   ON AN ELECTRICALLY - INSULATING        │────  410
│   SUBSTRATE                              │
└─────────────────────────────────────────┘
                    │
                    ▼
┌─────────────────────────────────────────┐
│   TREATING THE UPPER SURFACE OF          │
│   THE METAL ELECTRODES TO FORM           │────  420
│   HYDROPHILICITY - ENHANCING             │
│   MOIETIES THEREON                       │
└─────────────────────────────────────────┘
                    │
                    ▼
┌─────────────────────────────────────────┐
│   DEPOSITING AN ENZYMATIC                │
│   REAGENT LAYER ON THE                   │────  430
│   TREATED UPPER SURFACE                  │
└─────────────────────────────────────────┘
```

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5708247 A
- EP 1235068 A
- EP 0795601 A
- US 6241862 B
- US 6733655 B
- WO 0173109 A